(19) Europäisches Patentamt
European Patent Office
Office européen des brevets

(11) Publication number: 0 187 500 B1

# (12) EUROPEAN PATENT SPECIFICATION

(45) Date of publication of patent specification: 28.04.93

(51) Int. Cl.5: C07D 205/085, C07D 403/12, C07D 417/12, C07F 7/10, C07F 9/553, A61K 31/395

(21) Application number: 85309152.8

(22) Date of filing: 16.12.85

## (54) Monobactams.

(30) Priority: 20.12.84 US 683975
24.10.85 US 789158

(43) Date of publication of application:
16.07.86 Bulletin 86/29

(45) Publication of the grant of the patent:
28.04.93 Bulletin 93/17

(84) Designated Contracting States:
BE CH DE FR GB IT LI NL SE

(56) References cited:
EP-A- 0 061 765
EP-A- 0 076 758
EP-A- 0 091 239
EP-A- 0 093 376
EP-A- 0 111 326
DE-A- 2 748 258

J. Med. Chem. 26 (1983) 259

(73) Proprietor: THE UPJOHN COMPANY
301 Henrietta Street
Kalamazoo, Michigan 49001(US)

(72) Inventor: Kim, Kyoung Soon
The Upjohn Company 301 Henrietta Street
Kalamazoo Michigan 49001(US)
Inventor: Magerlein, Barney John
The Upjohn Company 301 Henrietta Street
Kalamazoo Michigan 49001(US)

(74) Representative: Perry, Robert Edward et al
GILL JENNINGS & EVERY, Broadgate House,
7 Eldon Street
London EC2M 7LH (GB)

EP 0 187 500 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel 2-oxoazetidine analogues (monobactams) having anti-microbial activity and to novel intermediates.

BACKGROUND

2-Oxoazetidine derivatives having anti-microbial and β-lactamase inhibitory activity are disclosed in EP-A-0053815, EP-A-0053816, EP-A-0076578 and EP-A-0096297. Each of these publications discloses β-lactams with various substituents at the C-4 position of the ring. For example, EP-A-0053816 generally describes the C-4 position as substituted by an organic residue.

Silyl-containing 2-oxoazetidine derivatives are disclosed in EP-A-0053387 and EP-A-0061765. 2-Oxo-1-(substituted phosphorus)azetidines are disclosed in US-A-4478749.

EP-A-0093376 discloses monobactams wherein the 4-position is substituted by a variety of residues containing nitrogen and oxygen atoms, inter alia, alkoxyalkyl, acyloxy, carbamoyl and carbamoyloxy. These compounds exhibit anti-microbial activity.

SUMMARY

Novel compounds according to the present invention are of formula I as defined in claim 1.

The amino-acid moiety, i.e. $-CO-(CH_2)_n-NR_2R_4$, is preferably in the alpha form.

When $R_4$ or $R_{11}$ is benzyloxycarbonyl (Boc), the compounds of formula I are intermediate compounds that are useful for preparing final compounds having anti-microbial activity. However, when Boc substituents are present, the resulting compounds do have some anti-microbial activity.

The term "substituted amino" (for $R_{20}$) refers to the radical $R_{21}$-NH- wherein $R_{21}$ is an acyl group derived from a carboxylic acid. A detailed description of the acyl groups included in $R_{21}$ can be found in U.S. Patent 4,478,749. The substituent $R_1$ in U.S. Patent 4,478,749 is the equivalent of $R_{21}$ herein and $R_1$ is described fully at column 8, line 41, through column 12, line 50, as those terms are defined in column 7, line 34, through column 8, line 22. The cited sections of U.S. Patent 4,478,749 are incorporated herein by reference.

The term "substituted amino" includes the above acylamino substituents where they may be optionally protected by conventional protecting groups known in the art. Examples of these protecting groups include t-butoxycarbonyl, phenylmethoxycarbonyl, phenylmethoxy or trityl and are used to protect amino and carboxyl groups found on acylamino substituents such as aminothiazole or oxyiminoacetic acid.

Preferred acyl groups of substituted amino $R_{20}$ include those which have been used to acylate 6-aminopenicillanic acid, 7-aminocephalosporanic acid and their derivatives which can be found in "Chemistry and Biology of β-Lactam Antibiotics," Vol. 3, Appendix A, R.B. Morin and M. Gorham, ed., Academic Press, N.Y. 1982 and include the following list of acylamino groups:

2-cyanoacetamido,
(aminophenylacetyl)amino,
[amino(4-hydroxyphenyl)acetyl]amino,
α(thien-2-yl)acetamido,
phenylacetamido,
(hydroxyphenylacetyl)amino,
[(formyloxy)phenylacetyl]amino,
[amino(4-hydroxyphenyl)acetyl]amino,
[[trifluoromethyl)thio]acetyl]amino,
2-(3,5-dichloro-4-oxo-1,4-dihydro-1(4H)-pyridyl)acetamido,
[(1H-tetrazol-1-yl)acetyl]amino,
[(2-amino-4-thiazolyl)(methoxyimino)acetyl]amino,
2-[cyanomethyl)thio]acetamido,
[[[(4-ethyl-2,3-dioxo-]-piperazinyl)carbonyl]amino](4-hydroxyphenyl)acetyl]amino,
[[2-(aminomethyl)phenyl]acetyl]amino,
4-(carbamoylcarboxymethylene)-1,3-dithiethane-2-carboxamido,
3-(o-chlorophenyl)-5-methyl-4-isoxazolecarboxamido,
2-[p-(1,4,5,6-tetrahydro-2-pyrimidinyl)phenyl]acetamido,
(amino-1,4-cyclohexadien-1-yl-acetyl)amino,
(phenylsulfoacetyl)amino,

(2R)-2-amino-2-(m-methanesulfonamidophenyl)acetamino,
[(2-amino-4-thiazolyl)(1-carboxy-1-methylethoxy)iminoacetyl]amino,
2-(1H-tetrazol-1-yl)acetamido,
[(2,3-dihydro-2-imino-4-thiazolyl)(methoxyimino)acetyl]amino,
2-(2-furyl)glyoxylamido,
(2-amino-4-thiazolyl)carboxy(methoxyimino)acetyl]amino.

The most preferred $R_{20}$ substituents are the substituted aminothiazolyl oximecarboxylic acids where the substituted oxime is in the syn configuration (Z).

A process for making compounds of Formula I comprises acylating compounds of Formula IV to make compounds of Formula IIb ($R_{10}$ = $-CO-(CH_2)_n-NR_2R_4$) which are then converted to compounds of Formula I. More particularly, a process is presented for modifying the $\beta$-lactam ring at the 3 and 4 carbon positions through the introduction of an alkyl or aryl silyl-protecting group on N-1.

## DETAILED DESCRIPTION

Unless otherwise indicated, the following terms are defined as follows:

Alkyl refers to an aliphatic hydrocarbon radical either branched or unbranched such as methyl, ethyl, n-propyl, isopropyl, n-butyl, isobutyl, sec-butyl, tert-butyl or the like.

Alkoxy refers to an alkyl radical which is attached to the remainder of the molecule by oxygen such as methoxy, ethoxy, isoproxy, n-butoxy, iso-butoxy, sec-butoxy, tert-butoxy or the like.

Aralkoxy refers to a radical in which an aromatic moiety is substituted for a hydrogen atom of an alkoxy such as phenylmethoxy or fluorenylmethyloxy and the like.

Formimino refers to a radical of the formula -CH(NH).

Guanyl refers to a radical of the formula $NH_2C(NH)$-

In the above description and throughout this document the parenthetical prefix ($C_n$-$C_m$) refers to the total number of carbon atoms in a generic carbon moiety following the prefix. The prefix is inclusive such that a ($C_1$-$C_4$)alkyl would include alkyls of one, two, three and four carbon atoms both straight and branched isomeric forms. Prefixes describing generic carbon moieties followed by carbonyl do not include the carbonyl among the carbon atoms limiting the total number possible. For example, ($C_1$-$C_4$)-alkoxycarbonylamino would include butoxycarbonylamino having a total of five carbon atoms.

The term "pharmaceutically acceptable salts" includes those compounds created by salt formation with the sulfo ($SO_3H$), or alkyl phosphono ($R_{11}$-$OPO_2H$) groups at the 1 position as well as those formed with the amino, amide or carboxyl moieties of substituents on positions C-3 and C-4. These include salts with acids, and with bases containing alkali metals or alkaline earth metals, and amines including ammonia.

Acid salts can be made by neutralizing the compounds described herein with the appropriate acid to below pH 7.0, and preferably to between pH 2 and 6. Suitable acids for this purpose include hydrochloric, sulfuric, phosphoric, hydrobromic, hydroiodic, acetic, lactic, citric, succinic, benzoic, salicyclic, pamoic, cyclohexansulfamic, methanesulfonic, naphthalenesulfonic, p-toluenesulfonic, maleic, fumaric, oxatic and the like.

Metal salts can be formed by dissolving the compounds in water and adding a dilute metal base until the pH is between 7 and 8. Metal salts include sodium, potassium, magnesium, aluminum and calcium salts. Amine salts, including those with organic bases such as primary, secondary, tertiary, mono-, di-, and polyamines can also be formed using the above-described or other commonly used procedures. Further, ammonium salts can be made by well-known procedures.

It will be apparent to those skilled in the art that compounds of Formulas I and II contain several chiral carbons. All of the racemic and optically active enantiomers of the compounds of Formula I are included within the scope of the invention. The invention also includes both the individual isomers and mixtures.

Specifically, the azetidinones of this invention have chiral carbon atoms at positions C-3 and C-4 of the $\beta$-lactam ring. The preferred form is cis at C-3 and C-4 and the most preferred form is 3(S) and 4(S) with regard to orientation at C-3 and C-4 provided that in compounds of Formula I where X is other than hydrogen that the relative priority between X and $R_{20}$ under the sequencing rules for absolute configuration remains unchanged from the relative priority when X is hydrogen. When the relative priority is changed with respect to $R_{20}$ and X, the most preferred form is that form in which $R_{20}$ is oriented on the same side of the $\beta$-lactam ring as 3(S), 4(S) compounds of Formula I where X is hydrogen. The phrase "C-3 and C-4 cis isomers" means that the substituents, $R_{20}$ and $-CH_2OCO(CH_2)_nNR_2R_4$ are both oriented on the same side of the $\beta$-lactam ring.

The compounds of formula I and their respective salts and acids have valuable antibiotic activity against a variety of gram-positive and gram-negative bacteria such as Escherichia coli, Klebsiella pneumoniae,

Pseudomonas aeruginosa, and Staphylcoccus aureus. The compounds may be used as medicaments for human beings or animals, as food preservatives in animal feed, or as microbial growth inhibitors in industrial mediums such as paint.

Compounds of the invention are tested for in vitro antimicrobial activity using standard testing procedures such as the determination of maximum inhibitory concentration (MIC) by agar dilution as described in "Methods for Dilution Antimicrobial Susceptibility Tests for Bacteria That Grow Aerobically" (MF-T) Published January 1983 by the National Committee for Clinical Laboratory Standards, 771 East Lancaster Avenue, Villanova, PA 19084. Briefly, MIC values are determined in unsupplemented Mueller Hinton Agar (MHA). The compounds tested are diluted serially into molten MHA at 47° C. The agar is poured into petri dishes and allowed to harden. The various bacterium used for testing are grown overnight on MHA at 35° C and transferred to Tryptiease Soy Broth (TSB) until a turbidity of 0.5 McFarland standard is obtained. The bacterium are diluted one to twenty in TSB and inoculated on the plates (1 $\mu$l using a Steers replicator). The plates are incubated at 35° C for 20 hours and the MIC is read to be the lowest concentration of drug that completely inhibits visible growth of the bacterium. The MIC test results of two compounds of this invention are found in Table 1.

The process for making compounds of Formula I is illustrated in Chart 1. In Chart 1 the starting compound IV, can be made by methods known in the art, i.e., .J. Am. Chem. Soc., 95:2401-2404 (1973); or by the novel process illustrated in Chart 2, as will be described below.

In step 1 of Chart 1, a compound of Formula IV is subjected to acylation to prepare a compound IIb. The general conditions for conducting this acylation are well known in the art. A preferred method involves reacting compound of Formula IV with the appropriate carboxylic acid in the presence of dicyclohexylcarbodiimide and 1-hydroxybenzotriazole and a catalytic amount of 4-dimethylaminopyridine. The reaction can be conducted at a temperature of about 0-30° C for a time of about 1 to 5 hours. The resulting compound of Formula IIb can be isolated from the reaction mixture by conventional means such as crystallization or chromatography and combinations thereof.

Step 2 involves the introduction of a sulfo or alkylphosphonic acid residue on to the nitrogen atom at position 1 of the azetidinone ring (IIb) to yield a compound of Formula I. This is accomplished by reacting the compound with a sulfonating or phosphorylating agent. Depending on the desired final product, protecting groups well known in the art are optionally placed on sites subject to random sulfonation or phosphorylation. Protecting groups include t-butoxycarbonyl, 9-fluorenylmethoxycarbonyl and phenylmethoxycarbonyl.

A preferred manner of introducing the sulfono group is by reacting dimethylformamide sulfur trioxide with an optionally protected compound of Formula IIb in the presence of a solvent. The reaction can be conducted at a temperature of about -20° to 60° C for a time of about 1 to 5 hours. Preferred reaction temperature and reaction times are about 0 to 25° C and 1 to 3 hours, respectively. Solvents that can be used include dimethylformamide and methylene dichloride. The preferred solvent is dimethylformamide.

The protected sulfonated compound is removed from the reaction mixture by conventional means, i.e., an extractive work-up procedure and then deprotected to yield compounds of Formula I.

Deprotection, when needed, can be accomplished by methods well known in the art such as treating the compound with an acid.

The sulfonate compound I can be removed from the reaction mixture and further purified by methods well known in the art.

The general methods of phosphorylation of azetidine rings are known. Koster, W.H. et al., J. Am. Chem. Soc. 105:3743 (1993).

A preferred method of phosphorylation is by treating the -2-azetidinone with an alkyllithium base (typically n-butyllithium) followed by alkylphosphorochloridate (typically methyl or n-butyl phosphorochloridate) at -78° C for 2-3 hours. The resulting dialkylphosphonate azetidinones are then dealkylated by methods known in the art. Gray, M.D.M. and Smith, D.J.H., Tet. Lett. 21:859 (1980). The preferred dealkylation is by treating the dialkylphosphonate azetidinones with thiourea in acetonitrite at reflux temperatures for 1-2 hours to obtain 2-azetidinone-1-phosphonic acid. Typical examples of phosphorylated azetidinones are cis-(±)-3-[2'-(2''-amino-4''-thiazolyl)-(Z)-2'-(1'''-carboxymethoxy)imino]acetamido-4-N-formylglycinoyl-oxymethyl-2-oxy-1-azetidinemethylphosphonic acid potassium salt and cis-(±)-3-[2'-(2''-amino-4''-thiazolyl)-(Z)-2'-(1'''-carboxymethoxy)imino]acetamido-4-N-formylglycinoyloxymethyl-2-oxy-1-azetidine-N-butylphosphonic acid potassium salt.

Compounds of Formula IV may be substituted at C-3 with X where X is hydrogen, methoxy or formylamino. Methods for introducing methoxy and formylamino substituents at C-3 of the $\beta$-lactam ring are known in the art. J. Am. Chem. Soc., 95:2401-2404 (1973).

As indicated above, some starting material utilised in the process illustrated in Chart 1, are prepared by the novel process illustrated in Chart 2 (wherein $R_{12}$, $R_{13}$ and $R_{14}$ are independently selected from ($C_1$-$C_4$) alkyl and phenyl).

The starting compound III is known. J. Org. Chem., 47:2765-2767 (1982). It may be cis or trans with respect to the substituents on C-3 and C-4. It is recognised that alternative alkoxycarbonyls of from 1-5 carbon atoms, inclusive, or aralkoxycarbonyls of from 5-14 carbon atoms, inclusive, either unsubstituted or substituted by 1-4 groups of halogen, nitro, or ($C_1$-$C_4$) alkyl could be used in place of the phenylmethoxycarbonyl group on compound III.

In Step 1, the nitrogen at position 1 is protected by conversion to a compound of Formula IIc wherein $R_{12}$-$R_{14}$ have the meanings defined in Formula II. Silylation is the preferred protection method. Silylating agents well known in the art may be used. Typically, a trialkylsilyl chloride or an arylalkylsilyl chloride in the presence of an organic base is used. The reaction is conducted at a temperature of about 0 to 25°C for a period of about 1 to 5 hours in any of several anhydrous solvents, e.g. ethyl acetate, dioxane, tetrahydrofuran, or dimethylformamide, in the presence of either an inorganic base or a tertiary amino such as trialkylamine or imidazole. A preferred solvent is dimethylformamide. Compounds of Formula IIc can be removed from the reaction mixture by conventional means such as crystallisation, filtration, chromatography and combinations thereof.

The protected amino azetidinone (IIc) is reduced in Step 2 to give a compound of Formula IId. The reaction is conducted in the presence of a metal hydride at a temperature range of 0°C to room temperature for times of 2 to 5 hours. The preferred method is to treat compounds of Formula IIc with lithium borohydride in anhydrous tetrahydrofuran under cold conditions for several hours. Compounds of Formula IId can be obtained from the reaction mixture by conventional methods such as crystallisation or column chromatography, and combinations thereof.

In Step 3, hydrogenolysis of the phenylmethoxycarbonyl substituent of compounds of Formula IId is carried out in the presence of palladium-black or palladium on a support such as carbon under hydrogen gas to give compounds of Formula IIe.

In step 4, amino compounds (IIe) are converted to amides of Formula IIf wherein $R_{20}$ has the meaning defined in Formula I. This conversion may be carried out by any of a number of amide or peptide forming reaction sequences such as described in Methoden der Organischem Chemie, Vierte Auflage, Band XV/2, E. Wunch ed., Georg Thieme Verlag, Stuttgart, p. 1. A preferred acylation process is the use of approximately molar quantities of a desired acid, 1-hydroxy-benzotriazole, and a carbodiimide, such as dicyclohexylcarbodiimide. There reagents are added to the solution of the amine in a solvent, such as tetrahydrofuran, dimethylformamide, or acetonitrile. A temperature of 0° C - 60° C is operative, with 20-35° C preferred. The time of reaction is variable from 0.5-24 hr being required, although usually 3-4 hr is sufficient. A precipitate of dicyclohexylurea is formed during the reaction. This is removed by filtration. The amides (IIf) are isolated from the filtrate by extractive procedures and chromatography.

In step 5, the silyl group of compounds of Formula IIf are removed to yield compounds of Formula IIg-(IV) which are compounds of Formula IV where X is hydrogen. Desilylation is accomplished by reacting compounds of Formula IIf with a fluoride ion in the presence of a solvent at a temperature of about -20° to 60°C for a time of about 1 to 12 hours. Preferred reaction temperature and times are about 0 to 25°C and 1 to 5 hours, respectively. Solvents that can be used include methylene dichloride, methanol, tetrahydrofuran and ethanol. The preferred method of conducting this step is to treat compounds of Formula IIf with triethylammonium fluoride in methanol. Compounds of Formula IIg(IV) are then used as starting material as illustrated in Chart I.

Optically active isomers of the disclosed compounds are resolved by methods known in the art. Takeda European patent application 8310461-3. The resolving agents are any of the known resolving agents such as optically active camphorsulfonic acid, bis-o-toluoyltartaric acid, tartaric acid, and diacetyl tartaric acid which are commercially available and which are commonly used for resolution of amines (bases), as for example in Organic Synthesis, Coll. Vol. V., p. 932 (1978), resolution of R-(+) and S(-)-$\alpha$-phenylethylamine with (-)-tartaric acid.

Compounds of this invention are converted into optically active diastereomeric salts by reaction with an optically active acid in a manner standard in the isomer resolution art. The optically active acids include the resolving agents described above. These diastereomeric salts can then be separated by conventional means such as differential crystallization. Diastereomeric salts have different crystallization properties, which are taken advantage of in this separation. On neutralization of each diastereomeric salt with aqueous base in the corresponding optically active enantiomers of Formula I compound are obtained, each of which can subsequently and separately be converted as herein described to the desired salt.

Preferably the separation of enantiomers is carried out by forming salts of optically active tartaric acid or derivatives thereof and taking advantage of the difference in solubility between the resulting diastereomers. Salt formation is carried out prior to acylation of the amino acid moiety at the C-4 position of the azetidinone ring. The preferred starting compound for making optically active compounds of Formula I, cis-(±)-1[2',4'-dimethoxyphenyl)methyl]-4-(methoxycarbonyl)-3-phenylmethoxycarboxyamido-2-azetidinone, is known. Chem. Pharm. Bull., 32:2646-2659 (1984). The C-3 protecting group is removed by hydrogenolysis to the corresponding free amine. An appropriate substituted tartaric acid enantiomer is then added such as (+)-di-p-toluoyl-D-tartaric acid and reaction conditions altered to facilitate precipitation of the appropriate azetidinone diastereomeric salt. The tartaric acid is removed by treating the compound with inorganic base such as sodium bicarbonate to obtain C-3 free amino-azetidinone.

The compounds of Formula I are effective for treating bacterial infections in animals, including humans.

Various compositions of the compounds of the present invention are presented for administration to humans and animals in unit dosage forms. The term "unit dosage form", as used in the specification, refers to physically discrete units suitable as unitary dosages for human subjects and animals, each unit containing a predetermined quantity of active material calculated to produce the desired pharmaceutical effect in association with the required pharmaceutical diluent, carrier or vehicle. The specifications for the novel unit dosage forms of this invention are dictated by and directly dependent on (a) the unique characteristics of the active material and the particular effect to be achieved and (b) the limitations inherent in the art of compounding such an active material for use in humans and animals, as disclosed in detail in this specification, these being features of the present invention. Examples of suitable unit dosage forms in accord with this invention are tablets, capsules, pills, suppositories, powder packets, wafers, granules, cachets, teaspoonfuls, tablespoonfuls, dropperfuls, ampoules, vials, aerosols with metered discharges, segregated multiples of any of the foregoing, and other forms as herein described.

For oral administration, either solid or fluid unit dosage forms can be prepared. For preparing solid compositions such as tablets, the compounds of Formula I are mixed with conventional ingredients such as talc, magnesium stearate, dicalcium phosphate, magnesium aluminum silicate, calcium sulfate, starch, lactose, acacia, methylcellulose, and functionally similar materials as pharmaceutical diluents or carriers. Capsules are prepared by mixing the compound with an inert pharmaceutical diluent and filling the mixture into a hard gelatin capsule of appropriate size. Soft gelatin capsules are prepared by machine encapsulation of a slurry of the compound with an acceptable vegetable oil, light liquid petrolatum or other inert oil.

Fluid unit dosage forms for oral administration such as syrups, elixirs, and suspensions can be prepared. The water-soluble forms can be dissolved in an aqueous vehicle together with sugar, aromatic flavoring agents and preservatives to form a syrup. An elixir is prepared by using a hydroalcoholic (ethanol) vehicle with suitable sweeteners such as sugar and saccharin, together with an aromatic flavoring agent.

Suspensions can be prepared with an aqueous vehicle with the aid of a suspending agent such as acacia, tragacanth, methylcellullose and the like.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filtered sterilized before filling into a suitable vial or ampoule and sealing. Advantageously, adjuvants such as a local anesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection is supplied to reconstitute the liquid prior to use. Parenteral suspensions can be prepared in substantially the same manner except that the compound is suspended in the vehicle instead of being dissolved and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

Additionally, a rectal suppository can be employed to deliver the active compound. This dosage form is of particular interest where an animal cannot be treated conveniently by means of other dosage forms, such as orally or by insufflation, as in the case of young children or debilitated persons. The active compound can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate. These rectal suppositories can weigh from about 1 to 2.5 gm.

An effective quantity of the compound is employed in treatment. The dosage of the compound for treatment depends on many factors that are well known to those skilled in the art. They include for example, the route of administration and the potency of the particular compound. A dosage schedule for

humans having an average weight of 70 kilograms is from about 50 to about 3,000 mg of compound in a single dose, administered parenterally or in the compositions of this invention, are effective for treating bacterial infections. More specifically, the preferred single dose is from about 100 mg to 2,000 mg of compound. The oral and rectal dose is from about 100 mg to about 4,000 mg in a single dose. The preferred single dose is from about 100 mg to about 2,000 mg of compound.

Without further elaboration, it is believed that one skilled in the art can, using the preceding description, practise the present invention to its fullest extent. The following detailed examples describe how to prepare the various compounds and/or perform the various processes of the invention and are to be construed as merely illustrative. Those skilled in the art will promptly recognize appropriate variations from the procedures both as to reactants and as to reaction conditions and techniques.

Preparation 1 cis-(t)-1-t-Butyldimethylsilyl-3-[[2'-(phenylmethoxy) carbonyl]-amino]-4-methoxycarbonyl-2-azetidinone.

To a solution of cis-(±)-4-(methoxycarbonyl)-2-oxo-3[[(phenylmethoxy)carbonyl]amino]-1-azetidine (III) (56.1 g, 0.2 mole), triethylamine (26.5 g, 0.26 mole) and 4-dimethylaminopyridine (3.3 g, 0.027 mole) in 300 ml of anhydrous dimethylformamide at 0 °C, t-butyldimethylsilylchloride (33.4 g, 0.22 mole) is added with stirring. After 30 minutes the reaction temperature is warmed to room temperature, at which temperature it is stirred for 3 hours. The precipitated solid is filtered and the filtrate solution is concentrated under reduced pressure. The residue is dissolved in ethyl acetate, washed with water, dried over anhydrous sodium sulfate, concentrated under reduced pressure to obtain the title compound (71.9 g, 0.183 mole). The crude solid product is used directly for the next step without any further purification.

Melting point: 115-117 °C.

$^1$H-NMR (δ, $CDCl_3$): 7.33, 5.39, 5.1, 4.26, 3.7, 0.9, 0.35, 0.25.

Preparation 2 cis-(±)-1-t-Butyldimethylsilyl-4-hydroxymethyl-3-[[2'-(phenylmethoxy)carbonyl]amino]-2-azetidinone.

Lithium borohydride (1.47 g) is added with stirring to a solution of cis-(±)-1-t-butyldimethylsilyl-3-[[2'-(phenylmethoxy)carbonyl]amino]-4-methoxycarbonyl-2-azetidinone (6.907 g, 17.5 mmole) in 50 ml of anhydrous tetrahydrofuran at 0 °C. The reaction mixture is allowed to stay at 0 °C for 4 hours and then is quenched by adding acetic acid (16 ml) solution in ethyl acetate (50 ml) slowly followed by aqueous sodium bicarbonate solution. The organic layer is washed with brine, dried over anhydrous sodium sulfate and concentrated under reduced pressure to obtain a viscous material. This crude product is passed through a silica gel column eluting with hexane/ethyl acetate (2:1), which affords the title product (5.15 g, 14 mmole).

$^1$H-NMR (δ, $CDCl_3$): 7.4, 6.05, 5.25, 3.85, 2.45, 0.9, 0.35, 0.25.

Preparation 3 cis-(±)-1-t-Butyldimethylsilyl-4-hydroxymethyl-3-amino-2-azetidinone.

To a solution of cis-(+)-1-t-butyldimethylsilyl-4-hydroxymethyl-3-[[2'-(phenylmethoxy)carbonyl]amino]-2-azetidinone, (5.0 g, 13.7 mmole) in 50 ml of methanol, palladium-black (2.5 g) in 20 ml of ethanol is added and hydrogenolysis reaction is carried out under one atmosphere of hydrogen gas at room temperature. The reaction is complete in 40 minutes. Toluene (10 ml) is added to the mixture and stirred for 5 minutes. The catalyst is filtered and washed with methanol. The filtrate solution is concentrated under reduced pressure to obtain 3.16 g (13.7 mmole) of the title compound. This material is used directly for the next step.

Preparation 4 cis-(±)-1-(t-Butyldimethylsilyl)-3-[2'-(2''-triphenylmethyl amino-4''-thiazolyl)-(Z)-2'-(methoxyimino)acetamido]-4-hydroxymethyl-2-azetidinone.

To a mixture of cis-(±)-1-t-butyldimethylsilyl-4-hydroxymethyl-3-amino-2-azetidinone (crude product 320 mg, 1.4 mmole), 2-(2'-triphenylmethylamino)-2-(methoxyimino) acetic acid (860 mg, 87% purity by weight, 1.7 mmole), 1-hydroxybenzotriazole (227 mg, 1.7 mmole) in 10 ml of methylene dichloride at 0 °C, dicyclohexylcarbodiimide (347 mg, 1.7 mmole) was added. After one hour the ice bath is removed and the reaction mixture is allowed to stay at ambient temperature overnight. The solid is filtered and washed with 30 ml of methylene dichloride. To the filtrate solution 10 ml of aqueous sodium bicarbonate solution is added, stirred for 20 minutes and then the organic layer is dried over anhydrous sodium sulfate and concentrated under reduced pressure. Preparative thin layer chromatography on silica gel developing with

3:1/hexane:ethyl acetate affords the title product (700 mg, 1.07 mmole).

$^1$H-NMR ($\delta$, $CDCl_3$): 7.25, 6.45, 5.4, 3.95, 4.35 - 3.7, 0.9, 0.35, 0.25.

Preparation 5 cis-(±)-3-[2'-(2''-triphenylmethylamino-4''-thiazolyl)-(Z)-2'-(methoxyimino)acetamido)-4-hydroxymethyl-2-azetidinone.

To a solution of cis-(±)-1-(t-butyldimethysilyl)-3-[2'(2''-triphenylmethyl amino-4''-thiazolyl)-(Z)-2'-(methoxyimino)acetamido]-4-hydroxymethyl-2-azetidinone (656 mg, 1 mmole) in 10 ml of methanol 1.5 ml of 1 M solution of aqueous triethylammonium fluoride in methylene dichloride is added at room temperature. After one hour solid sodium bicarbonate (100 mg) is added to the reaction mixture and then the reaction is concentrated under reduced pressure. Ethyl acetate (100 ml) and water (50 ml) are added to the residue and the organic layer is taken, dried over anhydrous sodium sulfate and concentrated under reduced pressure to obtain the title product (509 mg, 0.95 mmole). This material is pure enough for the next step.

$^1$H-NMR ($\delta$, $CDCl_3$): 7.25, 6.65, 6.4, 5.4, 3.95, 4-3.5, 8.73, 8.3, 7.32, 6.8, 5.76, 5.15, 4.75, 3.8, 3.75 - 3.25, IR (neat) $cm^{-1}$ 3250, 1750, 1640.

Example 1: cis-(±)-3-[2'-(2''-amino-4''-thiazolyl)-(Z)-2'-(methoxyimino) acetamido]-4-[N-acetylglycinoyl-oxymethy]-2-oxo-1-azetidine sulfonic acid potassium salt.

Step 1

A mixture of cis-(±)-3-[2'-(2''-triphenylmethylamino-4''-thiazolyl)-(Z)-2'-(methoxyimino)acetamido]-4-hydroxymethyl-2-azetidinone (542 mg, 1 mmole), 1-hydroxybenzotriazole (1.04 mmole), N-acetylglycine (180 mg, 1.54 mmole), dicyclohexylcarbodiimide (340 mg, 1.64 mmole) and small amount of 4A molecular sieve in dimethylformamide (5 ml) is stirred at room temperature for 2 days. The solid material is filtered, washed with 100 ml of ethyl acetate and the filtrate solution is stirred in the presence of aqueous sodium bicarbonate solution (300 mg of sodium bicarbonate in 25 ml of water) for 20 minutes. The organic layer is taken, dried over sodium sulfate and concentrated under reduced pressure. The residue is passed through a medium pressure silica gel column eluting with hexane/ethyl acetate (1:1) followed by hexane/ethyl acetate (1:2) and then ethyl acetate to obtain 360 mg of cis-(±)-3-[2'-2''-triphenylmethylamino-4''-thiazolyl)-(Z)-2'-(methoxyimino)acetamido]-4-N-acetylglycinoyl-oxymethyl-2-azetidinone.

$^1$H-NMR ($\delta$, $CDCl_3$): 7.9, 7.28, 7.0, 6.63, 5.3, 3.94, 3.75, 4.4 - 4.0, 1.92.

Step 2

A solution of dimethylformamide-sulfur trioxide complex in dimethylformamide (0.5 ml of 0.95 M) is added to a solution of cis-(±)-3-[2-(2''-triphenylmethylamino-4''-thiazolyl)-(Z)-2'-(methoxyimino)acetamido]-4-[N-acetylglycinoyl-oxymethyl]-2-azetidinone (300 mg, 0.46 mmole) in methylene dichloride (3 ml) at 0°C and it is allowed to stay at 0°C for 20 minutes. When thin layer chromatography shows some starting material left an additional 0.25 ml of dimethylformamide-sulfur trioxide complex solution is added and stirred at 0°C for 20 minutes. The reaction is quenched by adding aqueous potassium phosphate monobasic (150 mg in 5 ml water) followed by tetra-N-butylammonium hydrogen sulfate (340 mg). The sulfonated product is extracted by use of methylene dichloride (50 ml), dried over sodium sulfate and concentrated under reduced pressure. The material obtained is dissolved in 10 ml of 70% formic acid and after 4 hours detritylation is complete. The solid is filtered, washed with 70% formic acid (10 ml) and the filtrate solution is concentrated under reduced pressure. Potassium salt formation is made by passing this tetra-n-butylammonium salt through Dowex-$K^+$ resin and purification by HP-20 resin column chromatograph yielding 200 mg of the title product (Example 1).

1H-NMR ($CD_3OD$): 6.84, 5.35, 4.47, 3.96, 3.93, 1.98.

Example 2 cis-(±)-3-[2'-(2''-amino-4''-thiazolyl)-(Z)-2'-(methoxyimino)acetamido]-4-N-phenylmethoxycarbonyl glycinoyl-oxymethyl-2-oxo-1-azetidinesulfonic acid potassium salt.

The title compound is produced by following the steps in Example 1, substituting N-phenylmethoxycarbonyl glycine methyl ester for N-acetylglycine.

1H-NMR ($\delta$, $CD_3OD$): 7.3, 6.85, 5.4, 5.1, 4.8 - 4.3, 2.95.

Example 3 cis-(±)-3-[2'-(2''-amino-4''-thiazolyl)-(Z)-2'-(methoxyimino)acetamido]-4-[N-formylglycinoyloxymethyl]-2-oxo-1-azetidine sulfonic acid potassium salt.

The title compound is produced by following the steps in Example 1, substituting N-formylglycine for N-acetylglycine.

1H-NMR ($CD_3OD$): 8.1, 6.85, 5.35, 4.45, 4.05, 3.95, 4.6 - 4.4.

Examples 4 & 5 cis-(±)-[2'(2''-amino-4''-thiazolyl)-(Z)-2'-(1''-carboxymethoxy)imino]acetamido-4-[N-formylglycinoyloxymethyl]-2-oxo-1-azetidinesulfonic acid potassium salt (4) and cis-±-3-[2'-(2''-amino-4''-thiazolyl)-(Z)-2'-(1'''-carboxymethoxy)imino]acetamido-4-[N-formylsulfonic acid glycinoyl-oxymethyl]-2-oxo-1-azetidinesulfonic acid dipotassium salt (5).

Step 1

The process of Examples 4 and 5 involves the addition of 2-(2-t-butoxycarbonylaminothiazol-4-yl)-(Z)-2-(t-butoxycarbonyl)-(methoxyimino)acetic acid at C-3 of the azetidine ring. The chemical process for making this substituent is outlined below.

(a) Ethyl-2-(2-t-butoxycarbonylaminothiazo1-4-yl)-acetate

Ethyl 2-(2-aminothiazol-4-yl-acetate), Justus Lizbigs Ann. Chem., 261:30-31 (1891) (73.1 g, 0.39 mole) is added to molten di-tert-butyldicarbonate with stirring at about 72 ° C. The temperature is raised to 100 ° C by heating in an oil bath of the distillate which is formed. After 2 hours at 100 ° C, the reaction mixture is cooled and evaporated at 2-3 mm using a condenser cooled to -78 ° C. This residue is dissolved in 1 L of Skellysolve B and passed through a column of 3.2 kg of silica gel. The column is eluted with 6 L of Skellysolve B, 9 L of Skellysolve B-ethyl acetate (6:1) and finally with Skellysolve B ethyl acetate (3:1). Fractions of 1 L are collected and monitored by thin layer chromatography using chloroform-methanol (10:1) and Skellysolve B-ethylacetate (2:1). The yield of the title compound (a) is of sufficient quality to use in the next step. $^{13}C$ NMR ($Me_2CO-d_6$) $\delta$ 13.8, 27.7, 36.6, 60.4, 82.0, 109.5, 143.9, 152.7, 160.8, 169.7; FAB exact mass of [M• + H] + calcd. for $C_{12}H_{19}N_2O_4S$: 287.1065. Found: 287.1042.

(b) Ethyl-2-(2-t-butoxycarbonylaminothiazol-4-yl)-2-oxoacetate

Selenium dioxide (4.24 g, 38.25 mmol) is added to a solution of ethyl-2-(2-t-butoxycarbonylaminothiazol-4-yl)-acetate in 73 ml of dioxane. The solution is heated to reflux for 15 min and allowed to cool. The cooled reaction mixture is filtered through a pad of filter aid and the dioxane distilled off under vacuum. The residue is then chromatographed over 200 g of silica gel in a short column using Skellysolve B-ethyl acetate (4:1) followed by Skellysolve B-ethyl acetate (2:1) for elution. Fractions are combined on the basis of thin layer chromatography profile to give the title compound (b).

$^{13}C$ NMR ($Me_2CO-d_6$) $\delta$ 13.8, 27.7, 62.2, 82.5, 126.9, 144, 153, 162, 164; FAB exact mass of $[M^{\bullet} + K]^{+}$ calcd. for $C_{12}H_{16}N_2O_5SK$: 339.0417. Found: 339.0409.

(c) 2-(2-t-Butoxycarbonylaminothiazol-4-yl)-2-oxoacetic acid.

While cooling in an ice-water bath, 37.4 ml of N NaOH (37.4 mmole) is added to a solution of 5.62 g (18.7 mmole) of the ethyl-2(2-t-butoxycarbonylaminothiazol-4-yl)-2-oxoacetate (b) in 75 ml of 95% EtOH. The bath is then removed and the reaction mixture stirred at ambient temperature for 15 min. The ethanol is removed at 2-3 mm at a temperature less than 30 ° C. The residue is dissolved in 180 ml of $H_2O$ and extracted with 70 ml of ether. The aqueous solution to which chips of ice are added, is then acidified by the addition of N HCL and extracted several times with ether. The ether is dried and evaporated yielding the title compound (c). $^{13}C$ NMR ($Me_2CO-d_6$) $\delta$ 27.9, 81.8, 126.8, 143.3, 153.2, 160.7, 165.5. FAB exact mass of $[M^{\bullet} + H]^{+}$ calcd. for $C_{10}H_{13}N_2O_5S$: 273.0545. Found: 273.0529.

(d) O-[(t-butoxycarbonyl)methyl-hydroxylamine.

Triethylamine (11.13 g, 0.11 mol) at room temperature to a solution of N-hydroxyphthalimide (16.3 g, 0.1 mol) in 100 ml of dimethylformamide. t-Butyl bromoacetate is added dropwise and the reaction mixture warmed to 60 ° C for 3 hours. The reaction mixture is then poured into 350 ml of ice-water with stirring. The

resulting crystals are filtered and washed in water and then dried and recrystallized from 140 ml of ethyl acetate to give N-[t-butoxycarbonyl)methoxy)]phthalimide.

$^{13}$C NMR ($Me_2CO$-$d_6$ + DMSO-$d_6$) δ 27.6, 73.5, 81.9, 123.2, 128.5, 134.8, CO not observed. FAB exact mass of $[M^{\bullet} + H]^+$ calcd. for $C_{14}H_{16}NO_5$: 278.1028. Found: 278.1018.

A reaction mixture of N-[t-(butoxycarbonyl)methoxy)] phthalimide (2.77 g, 10 mmole) and hydrazine hydrate (1.10 g, 22 mmol) in 40 ml of methylene diohloride is heated under reflux for 1 hour and the mixture is filtered. The filtrate is washed with dilute ammonium hydroxide, twice with water and dried. The solvent is distilled and the residue stirred with 10 ml of ether and filtered. The solvent is evaporated from the filtrate and the resulting oil passed through a chromatography column of 35 g of silica gel (Skellysolve B - ethyl acetate 3:1) to yield the title compound (d).

$^{13}$C NMR ($CDCl_3$) δ 28.2, 72.9, 81.3, 170.0. Mass spectrum showed highest peak at m/z = 132 ($M^{\bullet}$-$CH_3$); $C_6H_{13}NO_3$ requires 147.

(e) <u>2-(2-t-Butoxycarbonylaminothiazol-4-yl)-(Z)-2-(t-butoxycarbonyl)(methoxyimino)acetic acid.</u>

O-[(t-butoxycarbonyl)methyl]-hydroxylamine (1.32 g, 8.98 mmole) and 2-(2-t-butoxycarbonylaminothiazol-4-yl)-2-oxoacetic acid (2.22 g, 8.16 mmole) are mixed in 8 ml of pyridine and 30 ml of 95% ethanol and stirred for 2 hours at ambient temperature. The solvent is distilled under vacuum and the residue dissolved in 40 ml of water. The water is extracted twice with 15 ml portions of ether. The aqueous solution is next cooled, 40 ml of ether added and acidified. The aqueous solution is extracted twice more with ether and the ether washed with water, dried and evaporated. The crystal residue is then titurated with 20 ml of ethyl acetate to yield the title compound (e).

$^{13}$C NMR ($Me_2SO_2$-$d_6$) δ 27.7, 72.3, 82.4, 114.3, 148, 152. 1H NMR (MeOD) δ 28.4, 72.7, 83.0, 115, 142, 154, 150, 170. FAB exact mass of $[M^{\bullet} + H]^+$ calcd. for $C_{16}H_{24}N_3O_7S$: 402.1335. Found: 402.1333. Anal. calcd for $C_{16}H_{23}N_3O_7S$: C, 47.87; H, 5.77; N, 10.47; S, 7.99. Found: C, 47.81; H, 5.81; N, 10.39; S, 8.11. mp 180-182 dec.

Step 2

To a mixture of cis-(±)-1-t-butyldimethylsilyl-4-hydroxymethyl-3-amino-2-azetidinone (3.6 g, 15.6 mmole), 2-(2-t-butoxycarbonylaminothiazole-4-yl)-(Z)-2-(t-butoxcarbonyl)(methoxyimino)acetic acid (6.9 g, 17.16 mmole) and 1-hydroxybenzotriazole (2.11 g, 15.6 mmole) in 40 ml of methylene dichloride in the ice bath, dicyclohexylcarbodiimide (3.86 g, 18.72 mmole) is added with stirring. The ice bath is removed after 2 hours and the reaction mixture is stirred overnight at room temperature. The precipitated solid is filtered off, washed with 60 ml of methylene dichloride and the filtrate solution is stirred with aqueous sodium bicarbonate (1.5 g sodium bicarbonate in 35 ml water) for 20 minutes at room temperature. The organic layer is taken, dried over sodium sulfate and concentrated under reduced pressure. Treatment of the residual material with ether yields the first crop of a white solid product (4.5 g). The mother liquor is concentrated and passed through the medium pressure silica gel column eluting with 3:1/hexane:ethyl acetate to obtain additional 2.2 g of cis-(±)-1-(t-butyldimethysilyl)-3-[2'-[(2''-t-butoxycarbonyl amino)-4''-thiazolyl]-[2'-(1'''-t-butoxycarbonylmethoxy)imino]]-acetamido-4-hydroxymethyl-2-azetidinone.

1H-NMR (δ, $CDCl_3$): 8.2, 8.0, δ 7.28, 5.5, 4.66 and 4.64, 4.0 - 3.7, 1.6, 1.49, 1.54, 0.96, 0.24, 0.22.

Step 3

To a solution of cis-(±)-1-(t-butyldimethylsilyl)-3-[2'[(2''-t-butoxycarbonyl amino)-4''-thiazolyl]-(Z)-[2'-(1'''-t-butoxycarbonylmethoxy)imino]]acetamido-4-hydroxymethyl azetidinone (6.5 g, 10.6 mmole) in 60 ml of methanol 12.7 mmole of triethylammonium fluoride in methylene dichloride (prepared by mixing triethylamine and 48% hydrofluoric acid in methylene dichloride in an ice bath) is added at room temperature with stirring and the solution is allowed to stay for 30 min. Sodium bicarbonate solid (4.2 g) is added to the reaction mixture and most of the methanol is evaporated under the reduced pressure and then 150 ml of methylene dichloride and 30 ml of water are added. The organic layer is taken, dried over sodium sulfate and concentrated under reduced pressure to obtain 5.2 of a white solid material. This solid was washed with diethyl ether and dried to afford 4.9 6 of cis-(±)-3-[2'[(2''-t-Butoxycarbonyl amino)-4''-thiazolyl]-2'[(1'''-t-butoxycarbonylmethoxy)imino]-acetamido-4-hydroxymethy]-2-azetidinone.

MP 195 °C (decomp.).

1H-NMR (δ, $CDCl_3$): 8.4, 8.2, 7.35, 6.45, 5.5, 4.68, 4.2 - 3.7, 1.6, 1.50, 1.44.

Step 4

To a mixture of cis-(±)-3-[2'[(2''-t-butoxycarbonylamino)-4''-thiazolyl]-(Z)-2'-[(1'''-t-butoxycarbonylmethoxy)imino]acetamido-4-hydroxymethyl]azetidinone (4.5 g, 9 mmole), 1-hydroxybenzotriazole (1.216g, 9 mmole), dimethylaminopyridine (122mg, 1 mmole), N-formylglycine (1.62 g, 15.75 mmole) and some 4A molecular sieves in 60 ml of methylene dichloride and 6 ml of dimethylformamide, dicyclohexylcarbodiimide (3.25 g, 15.75 mmole) is added with stirring at room temperature. The reaction is complete in 2 hours. The precipitate solid is filtered off, washed with methylene dichloride (50 ml) and the filtrate solution is stirred with aqueous sodium bicarbonate (1.89 g sodium bicarbonate in 40 ml water) at room temperature for 15 minutes. The organic layer is taken, dried over sodium sulfate and concentrated under reduced pressure. The residue is passed through the medium presure silica gel column eluting with 3:1 hexane/ethylacetate followed by ethyl acetate to obtain 3.6 g of cis-(±)-3-[2'-[(2''-t-butoxycarbonylamino)-4''-thiazolyl]-(Z)-2-[(1'''-t-butoxycarbonylmethoxy)imino]]-acetamido-4-N-formylglycinoyl-oxymethyl]-2-azetidinone.

MP 108-110 °C.

1H-NMR (δ, $CDCl_3$): 8.85, 8.55, 7.35, 7.25, 7.0, 5.35, 4.63, 4.5 - 4.0, 1.53, 1.45.

Step 5

Dimethylformamide-sulfur trioxide complex solution (2.85 ml of 0.904 mole solution) is added to a solution of cis-(±)-3-[2'-[2''-t-butoxycarbonylamino)-4''-thiazolyl]-(Z)-2'-[(1'''-t-butoxycarbonylmethoxy)imino]-]acetamido-4-N-formylglycinoyl-oxymethyl]-2-azetidinone (1.5 g, 2.56 mmole) in dimethylformamide (5 ml) at 0 °C and stirred for one hour at that temperature. The ice bath is removed and it is allowed to stay at ambient temperature for an hour. When thin layer chromatography still shows starting material left, an additional 1 ml of dimethylformamide-sulfur trioxide complex solution is added. After 45 minutes a small amount of starting material is still left but the reaction is quenched by adding aqueous potassium phosphate monobasic solution (690 mg potassium phosphate monobasic in 40 ml water) and methylene dichloride (50 ml) followed by tetra-N-butylammonium hydrogen sulfate (1.74 g). The organic layer is taken, dried over sodium sulfate and concentrated under reduced pressure. The residue is dissolved in 10 ml of methylene dichloride cooled to 0 °C and trifluoroacetic acid (15 ml) is added with stirring. The ice bath is removed after 2 minutes and the reaction mixture is allowed to stay at ambient temperature for 30 minutes. The reaction mixture is concentrated under reduced pressure, washed with hexane (50 ml x 5) to remove the residual trifluoroacetic acid and the residual material was again concentrated under reduced pressure. The residue is dissolved in methanol (10 ml) and passed through Dowex-$K^+$ followed by HP-20 resin which affords monopotassium salt (275 mg) and dipotassium salt (192 mg).

Example 4: 1H-NMR ($D_2O$, $Me_2SiCD_2CD_2COO\text{-}NA^+$ as a reference) 8.14, 7.0, 5.2, 4.7 - 4.53, 4.44, 4.08.

Example 5: 1H-NMR ($D_2O$, $Me_3SiCD_2CD_2COO\text{-}Na^+$ as a reference) 8.87, 7.0, 5.54, 4.7 - 4.5, 4.55, 4.35.

Example 6 cis-(±)-3-[2'[2''-amino-4''-thiazolyl)-(Z)-2'-(1'''-carboxymethoxy)imino)]-acetamido-4-[L-N-formylalanoyloxymethyl]-2-oxo-1-azetidinesulfonic acid potassium salt.

The title compound is made by a procedure similar to that described in Examples 4 and 5, substituting L-N-formylalanine for N-formylglycine.

1H-NMR (D20, $Me_3SiCD_2CD_2CO_2\text{-}Na^+$ as a reference) 8.2, 5.52, 4.65 and 4.58, 5 - 4.4, 1.39.

Example 7 cis(±)-3-[2'(2''-amino-4''-thiazolyl)-(Z)-2'-(1'''-carboxymethoxy)imino)]acetamido]-4-[D-N-formylalanoyloxymethyl]-2-oxo-1-azetidinesulfonic acid.

The title compound is made by a procedure similar to that described in Examples 4 and 5, substituting D-N-formylalanine for N-formylglycine.

1H-NMR ($D_2O$, $Me_3SiCD_2CD_2CO_2\text{-}Na^+$ as a reference) 8.1, 5.52, 4.7 - 4.2.

FORMULAS

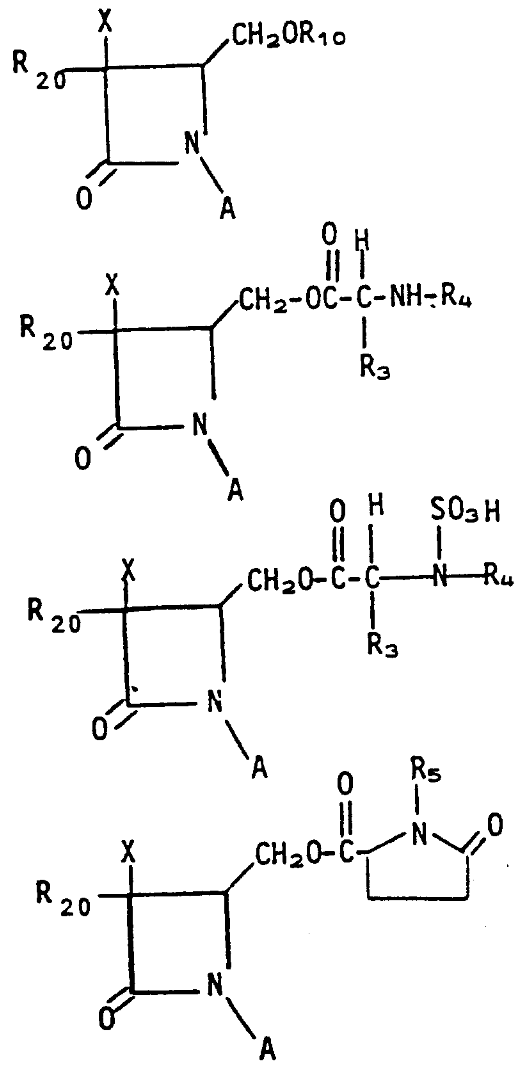

I

Ia

Ib

Ic

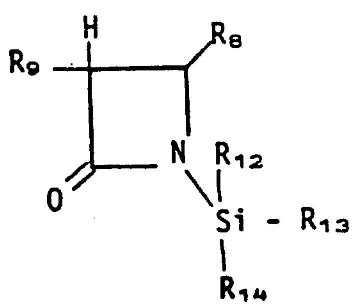

II

FORMULAS (Continued)

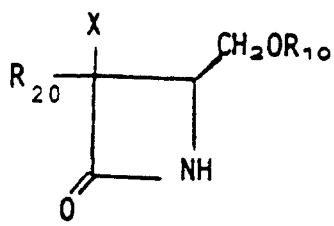

IIb

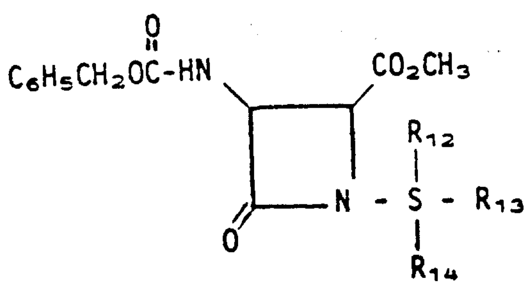

IIc

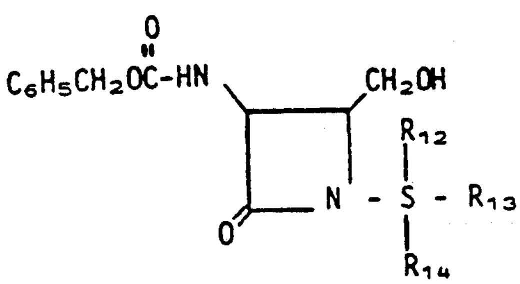

IId

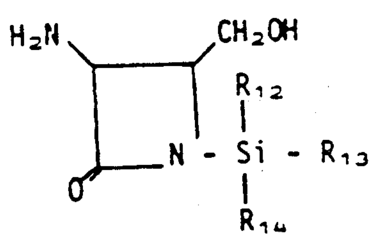

IIe

FORMULAS (Continued)

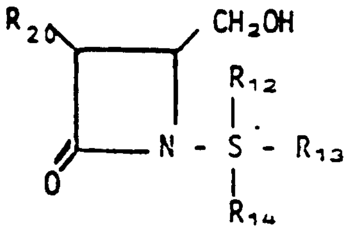

IIf

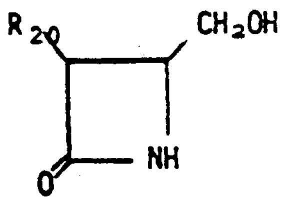

IIg(IV)

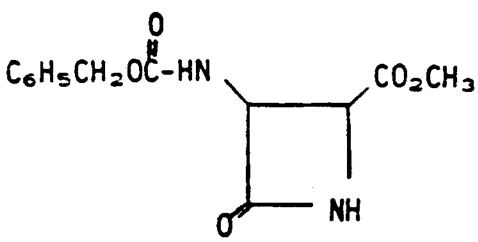

III

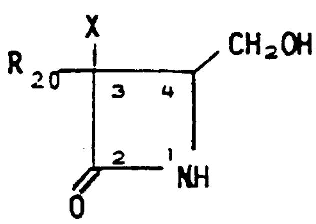

IV

CHART 1

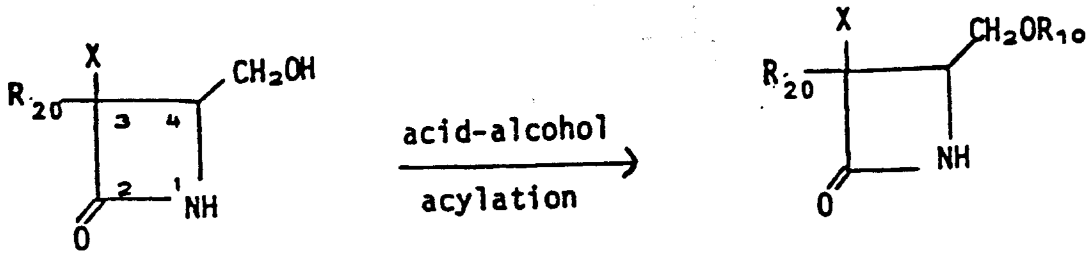


IV

IIb

IIb

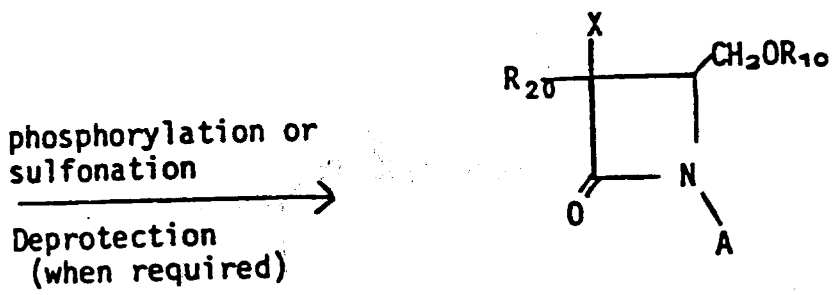


I

CHART 2

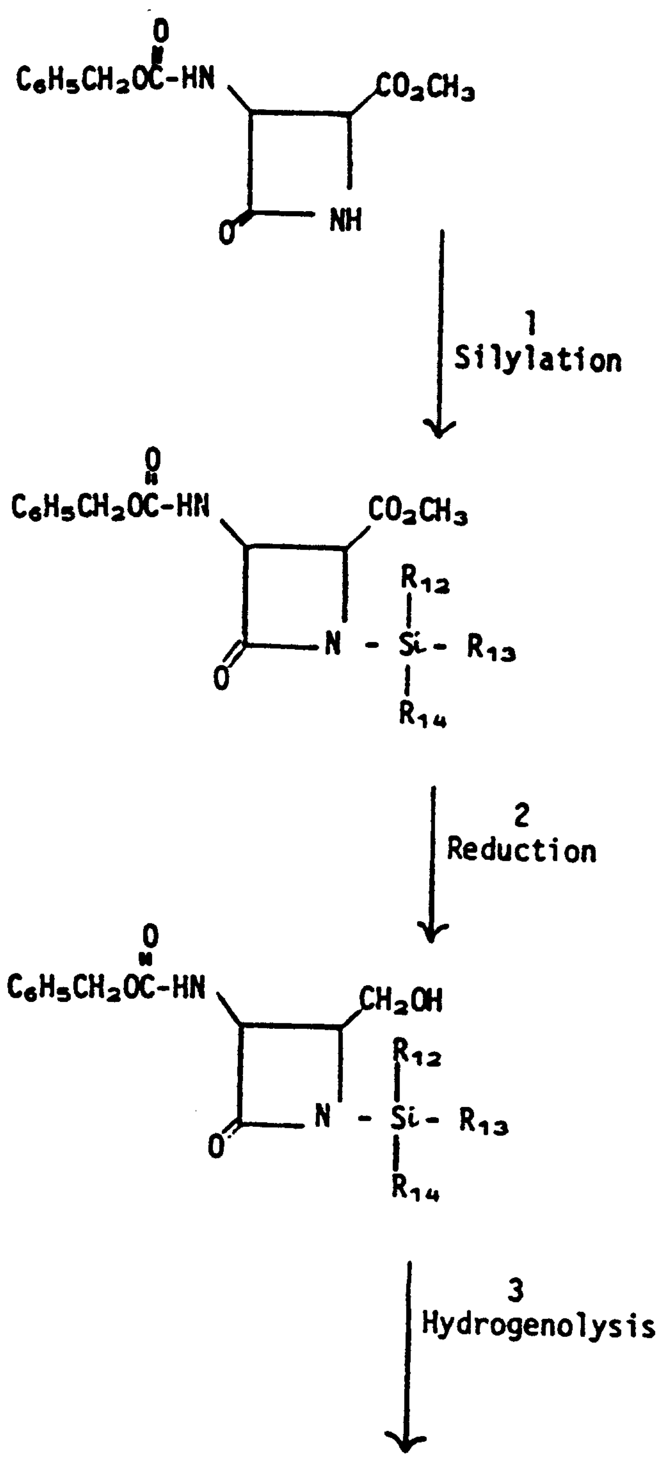


III

IIc

IId

CHART 2 (Continued)

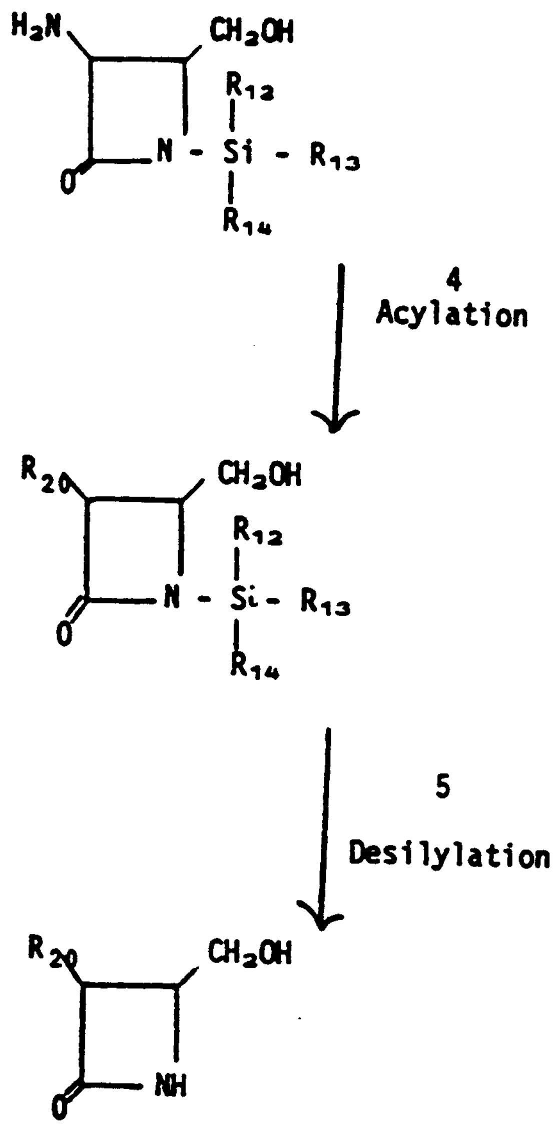


IIe

IIf

IIg(IV)

TABLE 1

ANTIMICROBIAL IN VITRO TESTING

Minimum Inhibitory Concentration -MCG per ML-

| Organism Name | Culture No. | Compound A[1] | Compound B[2] |
|---|---|---|---|
| Staphylococcus aureus | 6675 | >128 | >128 |
| Staphylococcus aureus | 3665 | >128 | >128 |
| Staphylococcus aureus | 6685 | >128 | >128 |
| Streptococcus faecalis | 694 | >128 | >128 |
| Streptococcus pneumoniae | 41 | 64 | 128 |
| Streptococcus pyogenes | 152 | 16 | 16 |
| Citrobacter freundii | 3507 | 0.125 | 0.25 |
| Enterobacter cloacae | 9381 | 64 | 64 |
| Enterobacter cloacae | 9382 | 0.125 | 0.25 |
| Escherichia coli | 311 | 0.06 | 0.125 |
| Escherichia coli | 9451 | 0.125 | ---- |
| Escherichia coli | 9379 | 0.125 | 0.25 |
| Escherichia coli | 9380 | 0.25 | 0.25 |
| Klebsiella oxytoca | 9383 | 0.5 | 1 |
| Klebsiella oxytoca | 9384 | 0.06 | 0.125 |
| Klebsiella pneumoniae | 58 | 0.06 | 0.125 |
| Proteus vulgaris | 9679 | <0.03 | 0.125 |
| Serratia marcescens | 6888 | 0.25 | 0.5 |
| Pseudomonas aeruginosa | 9191 | 4 | 8 |
| Pseudomonas aeruginosa | 6432 | 8 | 64 |
| Pseudomonas aeruginosa | 6676 | 4 | 8 |
| Pseudomonas aeruginosa | 30133 | 8 | 16 |

[1]Compound A is cis-(+)-[2'(2''-amino-4''-thiazolyl)-2'-(1'''-carboxymethoxy)imino]acetamido-4-N-formylglycinoyl-oxymethyl-2-oxo-azetidinesulfonic acid potassium salt.

[2]Compound B is cis-(+)-3-[2'-(2''-amino-4''-thiazolyl)-2'-(1'''-carboxymethoxy)imino]acetamido-4-N-glycinoyl-oxymethyl-2-oxo-1-azetidine sulfonic acid.

## Claims

**1.** An optically active compound, or a racemic mixture of compounds, of formula I

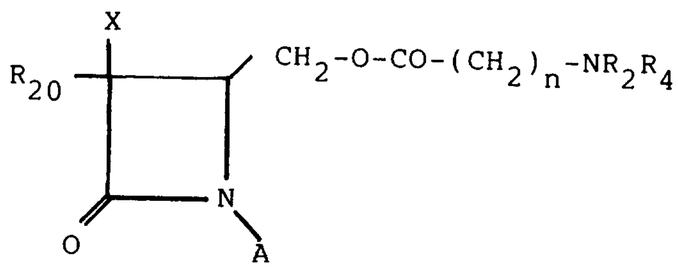

I

wherein n is one, 2, 3 or 4;

$(CH_2)_n$ is optionally substituted by a $C_{1-4}$ alkyl group itself optionally substituted by OH, COOH or SH;

A is $SO_3H$ or $P(O)(OH)R_{11}$ in which $R_{11}$ is $C_{1-4}$ alkyl, phenyl or benzyloxycarbonyl;

$R_2$ is H or as defined for A above;

$R_4$ is CH = NH, $C(NH_2) = NH$, benzyloxycarbonyl, CHO, ($C_{1-4}$ alkyl)carbonyl or carboxy($C_{1-4}$ alkyl)-carbonyl;

$R_{20}$ is amino or substituted amino; and

X is H, $OCH_3$ or CH = NH;

or a pharmaceutically acceptable salt thereof.

**2.** A compound of as claimed in claim 1, wherein n is one and $R_2$ is H or $SO_3H$.

**3.** A compound as claimed in claim 1, which is the (di)potassium salt of any C-3 or C-4 isomer of any of:

(a) 3-[2'-(2''-amino-4''-thiazolyl)-(Z)-2'-(methoxyimino)acetamido]-4-[N-(phenylmethoxycarbonyl)-glycinoyloxymethyl]-2-oxo-1-azetidinesulfonic acid,

(b) 3-[2'-(2''-amino-4''-thiazolyl)-(Z)-2'-(methoxyimino)acetamido]-4-(N-acetylglycinoyloxymethyl)-2-oxo-1-azetidinesulfonic acid,

(c) 3-[2'-(2''-amino-4''-thiazolyl)-(Z)-2'-(methoxyimino)acetamido]-4-(N-formylglycinoyloxymethyl)-2-oxo-1-azetidinesulfonic acid,

(d) 3-[[2'(2''-amino-4''-thiazolyl)-(Z)-2'-(1'''-carboxymethoxy)imino]acetamido]-4-(N-formylglycinoyloxymethyl)-2-oxo-1-azetidinesulfonic acid,

(e) 3-[[2'-(2''-amino-4''-thiazolyl)-(Z)-2'-(1'''-carboxymethoxy)imino]acetamido]-4-(N-formyl-N-sulfonateglycinoyloxymethyl)-2-oxo-1-azetidinesulfonic acid,

(f) 3-[[2'-(2''-amino-4''-thiazolyl)-(Z)-2'-(1'''-carboxymethoxy)imino]acetamido]-4-(L-N-formylalanoyloxymethyl)-2-oxo1-azetidinesulfonic acid, and

(g) 3-[[2'-2''-amino-4''-thiazolyl)-(Z)-2'-(1'''-carboxymethoxy)imino]acetamido]-4-(D-N-formylalanoyloxymethyl)-2-oxo-1-azetidinesulfonic acid.

**4.** A compound as claimed in claim 1, which is selected from:

(a) 3-[2'-(2''-triphenylmethylamino-4''-thiazolyl)-(Z)-2'-(methoxyimino)acetamido]-4-(N-acetylglycinoyloxymethyl)-2-azetidinone,

(b) 3-[[2'-(2''-t-butoxycarbonylamino)-4''-thiazolyl]-(Z)-2'-[(1'''-t-butoxycarbonylmethoxy)imino]-acetamido]-4-(N-formylglycinoyloxymethyl)-2-azetidinone,

(c) 3-[[2'-(2''-t-butoxycarbonylamino-4''-thiazolyl](Z)-2'-[(1'''-t-butoxycarbonylmethoxy)imino]-acetamido]-4-(D-N-formylalanoyloxymethyl)-2-azetidinone, and

(d) 3-[[2'-(2''-t-butoxycarbonylamino)-4''-thiazolyl]-(Z)-2'-[(1'''-t-butoxycarbonylmethoxy)imino]-acetamido]-4-(L-N-formylalanoyloxymethyl)-2-azetidinone.

**5.** A compound as claimed in any preceding claim, wherein the absolute configuration with respect to C-3 and C-4 is 3(S), 4(S).

**6.** A compound as claimed in any preceding claim, for therapeutic use.

## Patentansprüche

1. Optisch aktive Verbindung oder ein racemisches Gemisch von Verbindungen der Formel I

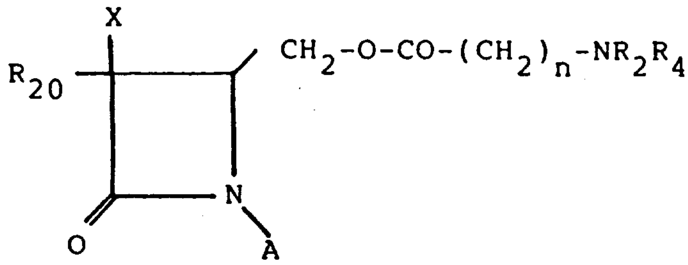

I

worin n = 1, 2, 3 oder 4;$(CH_2)_n$ gegebenenfalls durch eine ihrerseits gegebenenfalls durch OH, COOH oder SH substituierte $C_{1-4}$-Alkylgruppe substituiert ist;
A für $SO_3H$ oder $P(O)(OH)R_{11}$ mit $R_{11}$ gleich $C_{1-4}$-Alkyl, Phenyl oder Benzyloxycarbonyl steht;
$R_2$ H bedeutet oder entsprechend A definiert ist;
$R_4$ CH=NH, $C(NH_2)$=NH, Benzyloxycarbonyl, CHO, ($C_{1-4}$-Alkyl)carbonyl oder Carboxy($C_{1-4}$-alkyl)-carbonyl darstellt;
$R_{20}$ für Amino oder substituiertes Amino steht und X H, $OCH_3$ oder CH=NH entspricht,
oder ein pharmazeutisch akzeptables Salz hiervon.

2. Verbindung nach Anspruch 1, worin n = 1 und $R_2$ für H oder $SO_3H$ steht.

3. Verbindung nach Anspruch 1, in Form des (Di)-kaliumsalzes irgendeines C-3- oder C-4-Isomeren von:
   (a) 3-[2'-(2''-Amino-4''-thiazolyl)-(Z)-2'-(methoxyimino)acetamido]-4-[N-(phenylmethoxycarbonyl)-glycinoyl-oxymethyl]-2-oxo-1-azetidinsulfonsäure,
   (b) 3-[2'-(2''-Amino-4''-thiazolyl)-(Z)-2'-(methoxyimino)acetamido]-4-(N-acetylglycinoyloxymethyl)2-oxo-1-azetidin-sulfonsäure,
   (c) 3-[2'-(2''-Amino-4''-thiazolyl)-(Z)-2'-(methoxyimino)acetamido]-4-(N-formylglycinoyloxymethyl)2-oxo-1-azetidin-sulfonsäure,
   (d) 3-[[2'-(2''-Amino-4''-thiazolyl)-(Z)-2'-(1'''-carboxymethoxy)imino]acetamido]-4-(N-formylglycinoyloxymethyl)-2-oxo-1-azetidin-sulfonsäure,
   (e) 3-[[2'-(2''-Amino-4''-thiazolyl)-(Z)-2'-(1'''-carboxymethoxy)imino]acetamido]-4-(N-formyl-N-sulfonat-glycinoyloxymethyl)-2-oxo-1-azetidin-sulfonsäure,
   (f) 3-[[2'-(2''-Amino-4''-thiazolyl)-(Z)-2'-(1'''-carboxymethoxy)imino]acetamido]-4-(L-N-formylalanoyl-oxymethyl)-2-oxo-1-azetidin-sulfonsäure und
   (g) 3-[[2'-(2''-Amino-4''-thiazolyl)-(Z)-2'-(1'''-carboxymethoxy)imino]acetamido]-4-(D-N-formylalanoyl-oxymethyl)-2-oxo-1-azetidin-sulfonsäure.

4. Verbindung nach Anspruch 1, ausgewählt aus:
   (a) 3-[2'-(2''-Triphenylmethylamino-4''-thiazolyl)(Z)-2'-(methoxyimino)acetamido]-4-(N-acetylglycinoyl-oxymethyl)-2-azetidinon,
   (b) 3-[[2'-(2''-tert.-Butoxycarbonylamino)-4''-thiazolyl]-(Z)-2'-[(1'''-tert.-butoxycarbonylmethoxy)imino]-acetamido]-4-(N-formylglycinoyloxymethyl)-2-azetidinon,
   (c) 3-[[2'-(2''-tert.-Butoxycarbonylamino-4''-thiazolyl]-(Z)-2'-[(1'''-tert.-butoxycarbonylmethoxy)imino]-acetamido]-4-(D-N-formylalanoyloxymethyl)-2-azetidinon und
   (d) 3-[[2'-(2''-tert.-Butoxycarbonylamino)-4''-thiazolyl]-(Z)-2'-[(1'''-tert.-butoxycarbonyl methoxy)-imino]-acetamido]-4-(L-N-formylalanoyloxymethyl)-2-azetidinon.

5. Verbindung nach einem der vorhergehenden Ansprüche, wobei die absolute Konfiguration in Bezug auf C-3 und C-4 3(S), 4(S) ist.

6. Verbindung nach einem der vorhergehenden Ansprüche zum therapeutischen Gebrauch.

## Revendications

1. Composé optiquement actif, ou mélange racémique de composés, de formule I

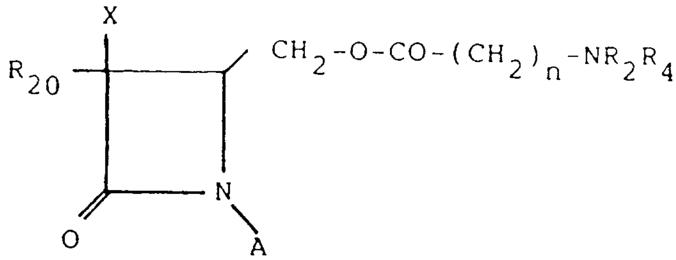

I

dans laquelle n est égal à un, 2, 3 ou 4 ;

le groupe $(CH_2)_n$ est facultativement substitué avec un groupe alkyle en $C_1$ à $C_4$, lui-même facultativement substitué avec un groupe OH, COOH ou SH ;

A représente un groupe $SO_3H$ ou $P(O)(OH)R_{11}$ dans laquelle $R_{11}$ représente un groupe alkyle en $C_1$ à $C_4$, phényle ou benzyloxycarbonyle ;

$R_2$ représente H ou répond à la définition précitée pour A ;

$R_4$ représente un groupe $CH=CH$, $C(NH_2)=NH$, benzyloxycarbonyle, CHO, (alkyle en $C_1$ à $C_4$)-carbonyle ou carboxy(alkyle en $C_1$ à $C_4$)carbonyle ;

$R_{20}$ représente un groupe amino ou amino substitué ; et

X représente H, un groupe $OCH_3$ ou $CH=NH$ ;

ou un de ses sels pharmaceutiquement acceptables.

2. Composé suivant la revendication 1, dans lequel n est égal à un et $R_2$ représente H ou un groupe $SO_3H$.

3. Composé suivant la revendication 1, qui est le sel (di)potassique de n'importe quel isomère C-3 ou C-4 de l'un quelconque des composés suivants :
   (a) l'acide 3-[2'-(2"-amino-4"-thiazolyl)-(Z)-2'-(méthoxyimino)acétamido]-4-[N-(phénylméthoxycarbonyl)glycinoyloxyméthyl]-2-oxo-1-azétidinesulfonique,
   (b) l'acide 3-[2'-(2"-amino-4"-thiazolyl)-(Z)-2'-(méthoxyimino)acétamido]-4-(N-acétylglycinoyloxyméthyl)-2-oxo-1-azétidinesulfonique,
   (c) l'acide 3-[2'-(2"-amino-4"-thiazolyl)-(Z)-2'-(méthoxyimino)acétamido]-4-(N-formylglycinoyloxyméthyl)-2-oxo-1-azétidinesulfonique,
   (d) l'acide 3-[[2'-(2"-amino-4"-thiazolyl)-(Z)-2'- (1'''-carboxyméthoxy)imino]acétamido]-4-(N-formylglycinoyloxyméthyl)-2-oxo-1-azétidinesulfonique,
   (e) l'acide 3-[[2'-(2"-amino-4"-thiazolyl)-(Z)-2'-(1'''-carboxyméthoxy)imino]acétamido]-4-(N-formyl-N-sulfonate-glycinoyloxyméthyl)-2-oxo-1-azétidinesulfonique,
   (f) l'acide 3-[[2'-(2"-amino-4"-thiazolyl)-(Z)-2'-(1'''-carboxyméthoxy)imino]acétamido]-4-(L-N-formylalanoyloxyméthyl)-2-oxo-1-azétidinesulfonique, et
   (g) l'acide 3-[[2'-(2"-amino-4"-thiazolyl)-(Z)-2'-(1'''-carboxyméthoxy)imino]acétamido]-4-(D-N-formylalanoyloxyméthyl)-2-oxo-1-azétidinesulfonique.

4. Composé suivant la revendication 1, qui est choisi entre :
   (a) la 3-[2'-(2"-triphénylméthylamino-4"-thiazolyl)-(Z)-2'-(méthoxyimino)acétamido]-4-(N-acétylglycinoyloxyméthyl)-2-azétidinone,
   (b) la 3-[[2'-(2"-t-butoxycarbonylamino)-4"-thiazolyl]-(Z)-2'-[(1'''-t-butoxycarbonylméthoxy)imino]-acétamido]-4-(N-formylglycinoyloxyméthyl)-2-azétidinone,
   (c) la 3-[[2'-(2"-t-butoxycarbonylamino-4"-thiazolyl]-(Z)-2'-[(1'''-t-butoxycarbonylméthoxy)imino]-acétamido]-4-(D-N-formylalanoyloxyméthyl)-2-azétidinone, et
   (d) la 3-[[2'-(2"-t-butoxycarbonylamino)-4"-thiazolyl]-(Z)-2'-[(1'''-t-butoxycarbonylméthoxy)imino]-acétamido]-4-(L-N-formylalanoyloxyméthyl)-2-azétidinone.

5. Composé suivant l'une quelconque des revendications précédentes, dans lequel la configuration absolue en rapport avec C-3 et C-4 est 3(S), 4(S).

6. Composé suivant l'une quelconque des revendications précédentes, destiné à une utilisation thérapeutique.